(19) Europäisches Patentamt
European Patent Office
Office européen des brevets

(11) **EP 4 157 409 B1**

(12) **EUROPEAN PATENT SPECIFICATION**

(45) Date of publication and mention of the grant of the patent:
**27.11.2024 Bulletin 2024/48**

(21) Application number: **21731303.0**

(22) Date of filing: **28.05.2021**

(51) International Patent Classification (IPC):
*A61M 16/00* (2006.01)   *A61L 9/20* (2006.01)
*A61M 16/06* (2006.01)   *A61M 16/08* (2006.01)
*A61M 16/10* (2006.01)   *A61M 16/12* (2006.01)
*A61L 2/00* (2006.01)

(52) Cooperative Patent Classification (CPC):
**A61M 16/0045; A61M 16/024; A61M 16/0627;
A61M 16/0816; A61M 16/0875; A61M 16/0891;
A61M 16/1005; A61M 16/125; A61M 16/127;**
A61L 9/20; A61M 16/0066; A61M 16/1065;
A61M 16/201; A61M 16/208; A61M 16/22;   (Cont.)

(86) International application number:
**PCT/IB2021/054704**

(87) International publication number:
**WO 2021/245516 (09.12.2021 Gazette 2021/49)**

(54) **ARTIFICIAL VENTILATION SYSTEM**

KÜNSTLICHES BEATMUNGSSYSTEM

SYSTÈME DE VENTILATION ARTIFICIELLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.06.2020 IT 202000013012**

(43) Date of publication of application:
**05.04.2023 Bulletin 2023/14**

(73) Proprietor: **WITA S.r.l.**
**38123 Trento (TN) (IT)**

(72) Inventors:
• **CONTI, Antonino**
**10126 Torino (IT)**
• **CONTI, Giuseppe**
**06000 Nice (FR)**
• **LASSOLA, Sergio**
**20871 Vimercate (MB) (IT)**
• **ZUCCO, Matteo**
**38060 Calliano (TN) (IT)**
• **CIANFLONE, Francesco**
**10098 Rivoli (TO) (IT)**

(74) Representative: **Bruni, Giovanni
Laforgia, Bruni & Partners
Corso Duca degli Abruzzi, 2
10128 Torino (IT)**

(56) References cited:
WO-A1-2011/030086   WO-A1-2012/103490
WO-A2-2007/030783   WO-A2-2007/128571
DE-A1- 3 536 519   US-A- 2 785 674
US-A1- 2008 078 393   US-A1- 2016 095 994
US-A1- 2019 167 928

(52) Cooperative Patent Classification (CPC): (Cont.)
A61M 2016/0027; A61M 2016/0033;
A61M 2016/1025; A61M 2016/103; A61M 2205/053;
A61M 2205/3368; A61M 2205/587; A61M 2230/005;
A61M 2230/06; A61M 2230/205; A61M 2230/42;
A61M 2230/432

**Description**

Technical field of the invention

The present invention relates to an artificial ventilation system.
In particular, the ventilation system is suitable for application to CPAP (Continuous Positive Airway Pressure) breathing helmets. By operating in this way, it is possible to provide artificial ventilation to a patient with respiratory difficulties destined for so-called "sub-intensive" therapies.

Background art

Breathing helmets of the type with continuous positive pressure mechanical ventilation are known, which allow to provide artificial ventilation to a patient with breathing difficulties. There are numerous models produced by different companies and all have an appearance reminiscent of a diver's helmet, in association with a plurality of tubes that carry oxygen and expel carbon dioxide. Known type helmets are completely transparent and fasten around the head with straps that can pass under the armpits. They are portable devices, sufficiently comfortable and light, which have a fairly low cost for a medical device. The internal volume is several liters (it allows the easy movement of the head) and the most comfortable models weigh a few hundred grams; they are all equipped with various safety systems, such as gauges to measure the internal pressure and anti-suffocation valves. Several clinical studies have shown its effectiveness in treating various conditions that determine respiratory failure, therefore CPAP helmets are versatile and potentially very useful devices to cope with the COVID-19 emergency. These tools are used for patients who require respiratory assistance but are not so severe that ICU (intensive care unit) admission is required.
US 2019/167928 discloses an artificial ventilation system.
Artificial ventilation systems of the known type, however, require very frequent checks by nurses or doctors, for their supervision and monitoring. The regulation of the flows of the respirators of the known systems occurs mostly according to manual control processes, thus requiring the almost constant presence of an operator, that is to say of specialized health personnel. Fully automatic and at the same time extremely safe and efficient artificial ventilation systems are not known.
Therefore, there is a need to define an artificial ventilation system and a related control method that are free from the aforementioned drawbacks.

Invention summary

An object of the present invention is to provide a fully automated artificial ventilation system which does not require frequent checks by specialized medical personnel.
Therefore, according to the present invention, an artificial ventilation system is provided with the characteristics set out in the attached independent product claim.
A control method of the artificial ventilation system is described, but not claimed.
This method allows automatic control of the entire artificial ventilation system and implements innovative control strategies.
Further preferred and / or particularly advantageous ways of implementing the invention are described according to the characteristics set forth in the attached dependent claims.

Brief description of the drawings

The invention will now be described with reference to the attached drawings, which illustrate some examples of non-limiting implementation of the housing element, in which:

- Figure 1 schematically shows an artificial ventilation system according to a first embodiment of the present invention,
- Figure 2 schematically shows an artificial ventilation system in a second embodiment of the present invention,
- Figure 3 is a logic diagram representing a control method of the artificial ventilation system according to Figures 1 and 2, in a first variant thereof,
- Figure 4 is a logic diagram representing a control method of the artificial ventilation system according to figures 1 and 2, in a second variant thereof, and
- Figures 5a, 5b and 5c show a detail of the ventilation system of figures 1 and 2.

Detailed description

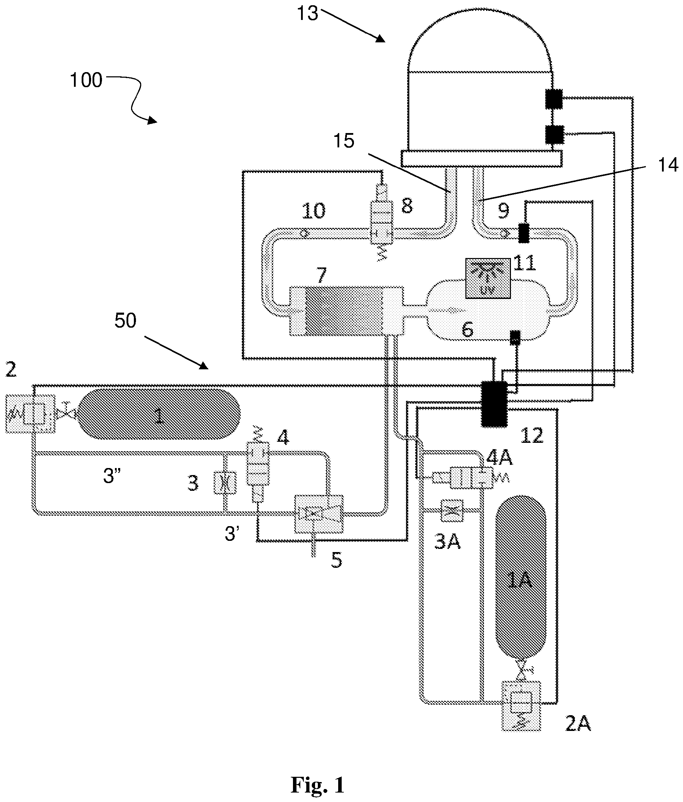

With reference now to Figure 1, a first embodiment of the artificial ventilation system 100 according to the present invention is described below by way of example only. The system comprises a respiratory helmet 13, for example, a respiratory helmet so-called CPAP (acronym from the English Continuous Positive Airway Pressure) of a known type and therefore not further described except for what will be said below about a device that can be implemented within it. The respiratory helmet 13, worn by a patient who needs artificial ventilation, will be fed by a mixture of air and oxygen and possibly also by a secondary medical gas, for example ozone by means of a supply duct 14, according to the hydraulic scheme that will be described, and the air containing carbon dioxide emitted by the patient will be expelled through the exhaust duct 15.

The supply of the air / oxygen mixture is carried out in a gas supply system 50. In particular, the supply is achieved thanks to a suitable suction means which, in the example of figure 1, is an ejector 5 which works by applying the known Venturi principle. The air is taken from the external environment while the oxygen is contained under pressure in a tank 1. Preferably, the tank 1 could be a compressed oxygen cylinder. A first control valve 2 regulates the mixing between air or oxygen and a first duct 3' and a second duct 3" branch off from it. The first duct 3' constitutes the supply line to the ejector 5, while the second duct 3" feeds the secondary flow to the ejector 5. As known, for the operation of the ejector according to the Venturi principle, the two flows at the inlet to the ejector 5 must have different pressures, since the fluid at a higher pressure will have to "drag" the fluid at a lower pressure, having a single flow at an intermediate pressure at the outlet of the ejector 5. The pressure difference between the two ducts 3' and 3" (and therefore between the two flows entering the ejector 5) is achieved by means of a calibrated orifice 3 interposed between the two ducts. A second control valve 4 determines the flow rate of the secondary flow of air and oxygen to the ejector 5. Thus, the concentration of oxygen in the air is determined by the first control valve 2 while the flow rate of the air / oxygen mixture is determined by the second control valve 4. The mixture of air and oxygen exiting the ejector 5, therefore after exiting the gas supply system 50, reaches a plenum 6, after passing through a filter element 7 which will be described below.

Advantageously, the plenum 6 can be provided with a light device 11 with ultraviolet radiation which guarantees the sterilization of the air / oxygen mixture, avoiding the spread of any contagion.

Preferably, the air-oxygen mixture could be integrated with the presence of a second medical gas, for example, ozone. In fact, as known, ozone is an excellent sanitizer to eliminate germs and bacteria. Its very high oxidizing power makes it an effective sanitizer and deodorant, certainly of interest in the current contingency of the fight against Covid 19. The supply of this secondary gas is very similar to that of the air / oxygen mixture. In fact, it will be sufficient to provide a tank 1A of this "Secondary Medical Gas" (SMG) under pressure and a circuit similar to that described for the air / oxygen mixture in which a first control valve 2A will be present to provide for the mixing of air and SMG, a calibrated orifice 3A to obtain the desired pressure drop and a second control valve 4A to regulate the flow rate of this second air / SMG mixture. The suction effect also for this second mixture will always be guaranteed by the ejector 5.

From the plenum 6, the air / oxygen mixture (possibly the air / oxygen / SMG mixture) reaches the respiratory helmet 13 by means of the supply duct 14, along which there is a first non-return valve 9 which prevents the backflow of the mixture. The mixture thus fed is then available for inspiration by the patient wearing the helmet 13.

Finally, the exhausted air, i.e. the air rich in carbon dioxide ($CO_2$) exhaled by the patient, is made to flow out by means of the exhaust duct 15 and the flow rate is regulated by a third control valve 8. A second non-return valve return 10 prevents the backflow of carbon dioxide towards the respiratory helmet 13. The air / $CO_2$ mixture is then filtered into the filter element 7 and the air thus purified from $CO_2$ rejoins the main flow of the air / oxygen mixture directed to the plenum 6.

The artificial ventilation system 100, as will be seen below, can be controlled automatically and for this purpose also includes a control unit 12 which can manage input data and control the appropriate actuators. The input data may be, according to a non-exhaustive list, the flow rate and pressure of the air / oxygen mixture (possibly of the air / oxygen / SMG mixture), the concentration of oxygen and $CO_2$ in the respiratory helmet 13, as well as pressure and temperature always inside the helmet. In addition, the $SpO_2$ oxygen saturation data is also fundamental, to which additional biomedical parameters, such as heart rate and respiratory rate, can be added. The control unit 12, according to strategies that will be illustrated below, will operate the first control valve 2 and the second control valve 4 to regulate the oxygen concentration and the flow rate of the air / oxygen mixture in the supply duct 14 (as well as, similarly the first control valve 2A and the second control valve 4A to regulate the concentration of SMG and the flow rate of the air / SMG mixture, if a second medical gas is present). The control unit 12 will also operate the third control valve 8 to adjust the exhaust air flow in the exhaust duct 15.

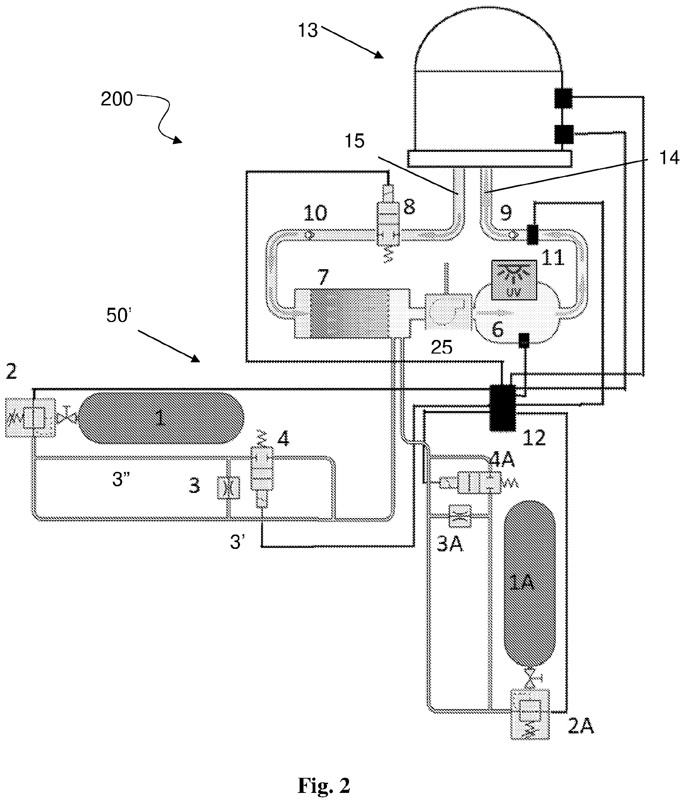

Figure 2 illustrates an artificial ventilation system 200 in a second embodiment of the invention. The ventilation system 200, as well as the previous one, includes the respiratory helmet 13. In practice, the ventilation systems referred to in Figures 1 and 2 differ only by the fact that in the diagram of Figure 2 there is a different gas supply system 50' which differs from the previous one only for the suction means which, according to this embodiment, is an electric fan 25. The fan creates an overpressure for the ventilation of the patient inside the helmet 13 and creates a low pressure for the intake of breathed air (rich in CO2) from the helmet. The choice of the suction means, ejector rather than fan or other similar means, will be dictated by the specific applications: the ejector, a static organ, may be more reliable, where an electric fan could guarantee greater flexibility within the required pressure head.

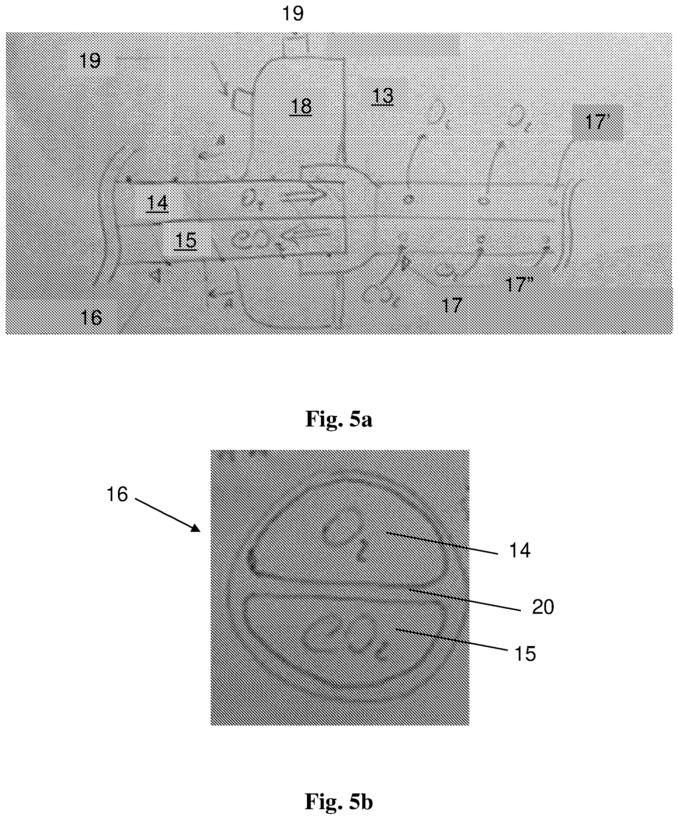

According to the present invention, the artificial ventilation system 100, 200 is provided with a particular layout involving the supply duct 14, the exhaust duct 15 and the respiratory helmet 13, as illustrated in Figures 5a to 5c.

In these Figures it can be seen that the supply duct 14 and the exhaust duct 15 are integrated in the first casing 16 which connects to a second casing 17 inside the respiratory helmet 13. The first casing 16 is in turn integrated in a coupling 18 which can be attached to the breathing helmet 13 by means of a bayonet connection or by means of threaded connections. Advantageously, the coupling 18 can be provided with sensors 19 in communication with the control unit 12 for the transmission of the parameters involved in the control strategies, as described hereafter.

The first casing 16, as mentioned above, groups together the supply duct 14 of the air / oxygen mixture (possibly air / oxygen / SMG) and the exhaust duct 15 of the exhausted air, rich in CO2. Inside the first casing 16 the two flows are evidently separated by means of a separation septum 20, as visible in Figure 5b. Similarly, also inside the second casing 17 the two flows will remain separated by a similar separation septum (not visible from the drawings). The second casing 17, inside the respiratory helmet 13, is provided with a double plurality of holes. A first plurality of holes 17' allows the air / oxygen mixture to escape inside the helmet 13, while the second plurality of holes 17" serves for the entry into the second casing 17 of the exhausted air, exhaled by the patient and therefore rich in CO2. The "flute" conformation of the second casing 17 is advantageous for a better circulation of the two flows (oxygen entering the helmet, CO2 exiting the helmet).

Furthermore, this layout has further advantages:

- the ability to customize the coupling 18 with all the necessary sensors depending on the specific application;
- the possibility of disassembling and sanitizing the coupling 18 and the relative sensors, where the respiratory helmet can be sanitized separately.

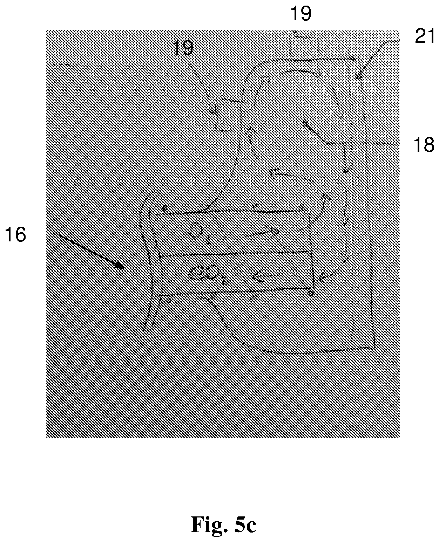

In particular, with reference to Figure 5c, the sanitization of the coupling 18 and of the sensors 19 is carried out by removing the coupling 18 from the respiratory helmet 13, sealing it with a suitable cap 21 and leaving the artificial ventilation system in operation. In this way, the mixture of air and oxygen will continue to circulate sterilizing the sensors thanks to its passage through the light device 11 with ultraviolet radiation.

As anticipated, the artificial ventilation system 100, 200 according to any of the embodiments described above can be managed and controlled automatically by means of the control unit 12.

A first control method 300 of the artificial ventilation system has two priority levels and is based on a closed loop control.

According to a first and most important priority level, the control unit maintains the patient's oxygen saturation SpO2 at the desired level, i.e. above a first threshold value and, at the same time, limits the CO2 concentration to helmet interior below a second threshold value.

With a second priority level, the control method optimizes the consumption of the externally supplied oxygen as well as patient comfort by keeping the air / oxygen mixture entering the helmet and the helmet pressure and temperature in corresponding predetermined intervals.

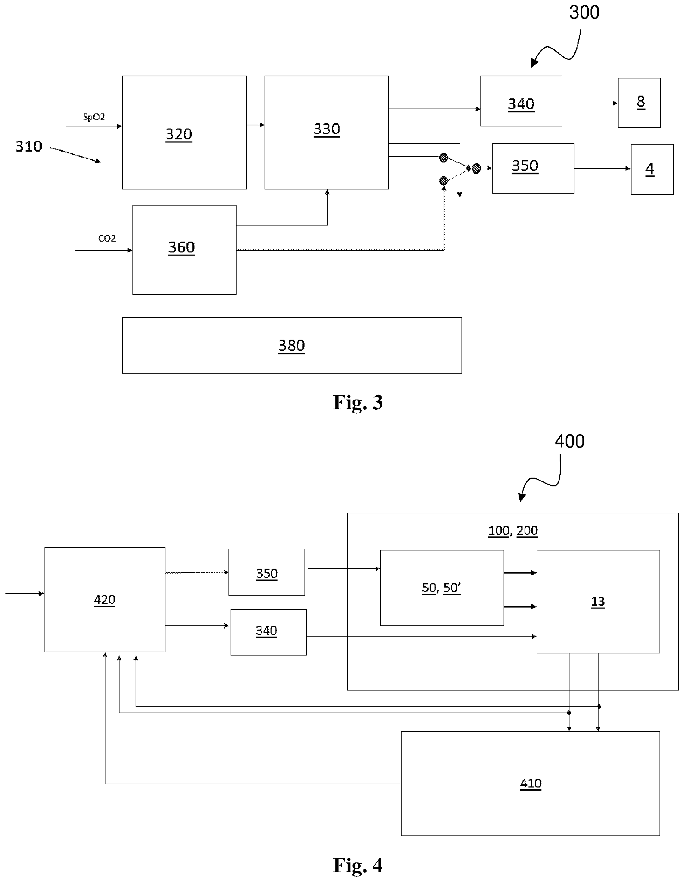

The first control method 300 is illustrated in Figure 3 which represents a block diagram thereof. A first control line 310 has as its objective the complete automation of the management of the respiratory helmet 13 according to the first priority level.

Control line 310 includes a closed-loop controller 320, such as a proportional-integrative-derivative (PID) controller, which adjusts the patient's oxygen saturation by comparing the input SPO2 saturation (the current value) with the desired level, above a first threshold value; the regulator 330 which manages the sequence of the implementation of the control valves; the actuators 340 and 350 enslaved to the regulator 330 which receive the logic command from the regulator 330 and give an electric command, respectively, to the third control valve 8 which regulates the flow rate of the outgoing flow from the helmet 13 (and therefore the CO2 flow rate) and to the second control valve 4 which regulates the flow of the air / oxygen mixture; the CO2 controller 360 that manages and monitors the amount of CO2 inside the helmet; a logic switch 370 which alternately connects the actuator 350 with the regulator 330 or with the CO2 controller 360.

Once the PID controller 320 has calculated the desired SPO2 value, the PID controller 320 communicates such a value to the regulator 330 which sets the sequence of the control valve actuation, which can be in the following order: first regulation of the exhaust air flow (containing CO2) by means of the actuator 340 which activates the third control valve 8 and subsequently, if the room for maneuver on the exhaust air flow control is ended (in other words, if the third control valve 8 is in an extreme position and is not further adjustable), regulation of the flow of the air / oxygen mixture by means of the actuator 350 which operates the second control valve 4.

This strategy is active, as described, when the CO2 concentration value remains below a second threshold value, monitoring chaired by the CO2 controller 360. Should the CO2 concentration exceed the second threshold value, the CO2 controller 360 has the authority to reverse the control valve actuation logic. In particular, the regulator 330, "ordered" by the CO2 controller 360, will use the logic switch 370 to enslave the actuator 350 to control the CO2 concentration. The actuator 350 will then actuate the second control valve 4 to regulate (in this case increase) the flow of the air / oxygen mixture inside the respiratory helmet 13. The closed-loop control of the patient's oxygen saturation remains active by means of the PID controller 320, but in this case the regulator 330 will only manage the actuator 340 which operates the third control valve 8 to regulate the exhaust air flow.

As mentioned, a second control line 380 with lower priority can be dedicated to the patient's comfort and contains suitable algorithms for monitoring some of his biomedical parameters. The second control line 380 proceeds in parallel with respect to the first control line 310, of higher priority, obviously subordinated to the correct operation of the parameters controlled by the first control line 310.

With reference to Figure 4, a second variant of the control method of the artificial ventilation system 100, 200 is now described.

The second control method 400 uses a dynamic model of the process to predict its future evolution and choose the best control action.

Also in this second case, the control method of the artificial ventilation system has two priority levels:

- first priority level. The control method maintains the patient's SpO2 oxygen saturation at the desired level, i.e. above a first threshold value and, at the same time, limits the CO2 concentration inside the helmet to below a second threshold value;
- second priority level. The control method optimizes externally supplied oxygen consumption and patient comfort, keeping the air / oxygen mixture entering the helmet and helmet pressure and temperature in corresponding predetermined ranges, as well as additional parameters related to the functioning of the control itself.

The block diagram of figure 4 shows:

- the controller 420 which is based on a predictive model able to describe some aspects of the ventilation system to be controlled and on other input parameters that will be defined below and can optimize a multivariable objective function with the boundary conditions that will be defined below. The controller 420 will directly operate the actuators that manage the oxygen flow and the CO2 concentration;
- the actuators 340 and 350 enslaved to the controller 420 which receive the logic command from the controller 420 and give an electrical command, respectively, to the third control valve 8 near the respiratory helmet 13 and to the second control valve 4 of the gas supply system 50, 50' to adjust the flow rate out of the helmet 13 (and therefore the CO2 flow rate) and the flow of the air / oxygen mixture;
- a state observer 410 which, in particular, provides for the estimate of physiological parameters of lung functions and forwards them to the controller 420.

More specifically, the state observer 410 receives as input data (see Figure 4):

A: parameters selected from an existing database. For example, SpO2 oxygen saturation, respiratory rate, heart rate, etc. The pre-existing database is a collection of human physiological parameters, grouped and clustered, the selection of which will be made on the basis of similarity between a patient and a specific cluster.
B: parameters measured in the respiratory helmet 13 thanks to the presence of suitable sensors. For example, pressure, temperature, oxygen concentration, air / oxygen mixture flow, CO2 concentration, etc.

The estimation model implemented in the state observer 410 allows instead to have as output data, by way of example, the models of the following physiological parameters of lung functions:
C: flows of oxygen and CO2 in the pulmonary alveoli and blood compartments, instantaneous volume of the lungs, pressure in the alveoli, etc.

The parameters indicated with A, B and C constitute input data for the controller 420. In addition, the controller 420 can manage:

D: parameters defined by the operator of the ventilation system. For example, the weights of the variables that realize the objective function to be optimized;
E: manipulable parameters. For example, the position, at a given instant of time, of the second control valve 4 and of the third control valve 8.

Based on all parameters A, B, C, D and E, the controller 420 is able to calculate the optimal values of oxygen saturation and CO2 concentration and to predict the evolution of the model results over a time interval defined by a number N of time steps, using a predetermined sequence of manipulable parameters E within the same time interval.

The algorithm is based on the optimization of an objective function, which evaluates the distance of the control system from the objectives and optimal control constraints, minimizing this distance. The control objectives are assessed in the predetermined time interval in which appropriate penalties are defined on the ability of the control system to follow its objectives and on the effort to implement the control valves.

An example of an objective function structured in this way is the following:

$$\sum_{k=0}^{N-1} w1 \ (100 - SpO2_k)^2 + w2 \ (Max \ CO2 - CO2_k)^2 +$$

$$w3 \ (valve \ 4 \ command)_k^2 + w4 \ (valve \ 8 \ command)_k^2$$

where:

- SpO2 is the oxygen saturation value,
- Max CO2 is the predetermined maximum concentration threshold of CO2,
- valve 4 command and valve 8 command represent the activity of the commands imposed, respectively, on the second control valve 4 and on the third control valve 8,
- w1, w2, w3, w4 are respective weights of the terms of the objective function, which can be set by the user. In practice, the parameters defined as D.

In other words, the objective function tends to make the oxygen saturation reach its maximum value of 100, as well as to keep the CO2 concentration below its maximum threshold. These two objectives represent, as in the case of the first control method 300, the first priority level.

According to a lower priority level, the optimization process of the objective function will have to take into account further control constraints related to:

- activity of the commands on the control valves 4, 8. In practice, the objective function will try, as far as possible, to also minimize the variation in the opening degree of the second control valve 4 and of the third control valve 8,
- maintaining the flow rate of the air / oxygen mixture and the temperature inside the helmet within predetermined intervals.

At each predetermined time instant t, the controller 420 will find the optimal sequence of manipulable parameters E (position of the second 4 and third control valve 8) which minimize the objective function in the time interval N. Obviously, only the first optimum activation will be used, then the controller 420 will act on the regulation of the exhaust air flow by means of the actuator 340 which activates the third control valve 8 and on the regulation of the air / oxygen mixture flow by means of the actuator 350 which activates the second control valve 4.

The procedure will be repeated at the next time instant t + 1.

As seen, the first control method 300 and the second control method 400 both derive from a single methodology which presents a strategy based on at least two priority levels. They are declined according to different approaches and can be used alternatively depending on the applications.

The first control method 300 is certainly easier to be implemented, does not require specific modeling (for example, mathematical models of lung functions) and does not require high computational skills.

The second control method 400 is more complex, requiring sophisticated modeling of lung functions and higher computational capacity. On the contrary, however, it allows in a single framework to monitor and assist patients with severe respiratory problems. Furthermore, due to its predictive approach and estimating the internal states of respiratory functions, the second control method is faster in reacting to any worsening of the patient's situation. Finally, it is more adaptable to a wider class of pathologies.

The control method 300, 400 for the artificial ventilation system 100, 200 may undergo further variations.

For example, it may also be able to control a mixture consisting of air / oxygen / SMG, in this case also acting on the control valve 4A of the air / SMG mixture.

Ultimately, the artificial ventilation system object of the present invention has undoubted advantages: it does not require any fixed infrastructure, since only needs the respiratory helmet, at least one cylinder for compressed oxygen, the electro-hydraulic circuitry, the ultraviolet light device and the control electronics. The artificial ventilation system is therefore completely portable and has ample flexibility for any application. Furthermore, the system is fully automatically controllable, with control strategies also based on sophisticated predictive algorithms, and does not require the presence on site of a health worker.

Although the above description makes it possible for the skilled person to implement the present invention at least according to an exemplary configuration thereof, it must be understood that numerous variations of the components described are conceivable, without thereby departing from the object of the invention, as defined in the appended claims, interpreted literally and / or according to their legal equivalents.

**Claims**

1.   Artificial ventilation system (100, 200) comprising:

- a respiratory helmet (13),
- suction means (5, 25) which draw the air from the external environment,
- a tank (1) for containing pressurized oxygen,
- a first control valve (2) which regulates the mixing of air/oxygen,
- a second control valve (4) which regulates the flow rate of the air/oxygen mixture,
- a plenum (6) for containing the mixture of air and oxygen leaving the suction means (5),
- a supply duct (14) which allows the air/oxygen mixture to reach the respiratory helmet (13),
- a first non-return valve (9) which prevents the backflow of the air/oxygen mixture from the supply duct (14),
- an exhaust duct (15) for the air/CO2 mixture,
- a filter element (7) of the air/CO2 mixture in fluid communication with the plenum (6), and
- a control unit (12) to control at least oxygen saturation and carbon dioxide concentration inside the respiratory helmet (13);

the artificial ventilation system (100, 200) being **characterized in that**:

- the air/CO2 mixture flow rate in the exhaust duct (15) is regulated by a third control valve (8),
- the supply duct (14) and the exhaust duct (15) are integrated in a first casing (16) which connects to a second casing (17) inside the respiratory helmet (13),
- wherein inside the first casing (16) and the second casing (17) the two flows remain separated by means of a separation septum (20), and
- the second casing (17) comprises a first plurality of holes (17') which allows the air/oxygen mixture to flow inside the respiratory helmet (13) and a second plurality of holes (17") for the entry of the exhausted CO2-rich air into the second casing (17).

2.   Artificial ventilation system (100, 200) according to claim 1, wherein the first casing (16) is integrated in a coupling (18).

3.   Artificial ventilation system (100, 200) according to claim 2, wherein the coupling (18) is equipped with sensors (19) in communication with the control unit (12).

4.   Artificial ventilation system (100, 200) according to any of the preceding claims, wherein the plenum (6) comprises an ultraviolet light device (11) for sterilizing the air/oxygen mixture.

5.   Artificial ventilation system (100, 200) according to any of the preceding claims, wherein:

- the suction means is an ejector (5) fed by a main flow coming from a first duct (3'), located downstream of the first control valve (2), and from a secondary flow coming from a second duct (3"), parallel to the first duct (3') and also located downstream of the first pilot valve (2), and
- a calibrated orifice (3) interposed between the two ducts (3', 3") creates a pressure difference between the secondary flow and the main flow.

6.   Artificial ventilation system (200) according to any of claims 1 to 4, wherein the suction means (5) is an electric fan (25) which creates an overpressure for ventilation inside the respiratory helmet (13) and a low pressure for suction of the air breathed by the respiratory helmet (13).

7.   Artificial ventilation system (100, 200) according to claim 1, wherein a second air/medical gas mixture is added to the air/oxygen mixture and the artificial ventilation system (100, 200) comprises a second tank (1A), a first control valve (2A) to mix the air and the medical gas, a second calibrated orifice (3A) to obtain a pressure drop and a second control valve (4A) which regulates the flow rate of this second air/medical gas mixture.

**Patentansprüche**

1. Künstliches Beatmungssystem (100, 200), umfassend:

   - einen Atemhelm (13),
   - Saugmittel (5, 25), die die Luft aus der Umgebung ansaugen,
   - einen Tank (1) zur Aufnahme von unter Druck stehendem Sauerstoff,
   - ein erstes Steuerventil (2), das die Mischung von Luft/Sauerstoff regelt,
   - ein zweites Steuerventil (4), das die Strömungsrate des Luft/Sauerstoffgemischs regelt,
   - ein Plenum (6) zur Aufnahme des Luft-Sauerstoffgemischs, das das Saugmittel (5) verlässt,
   - einen Zufuhrkanal (14), der es dem Luft/Sauerstoffgemisch ermöglicht, den Atemschutzhelm (13) zu erreichen,
   - ein erstes Rückschlagventil (9), das den Rückfluss des Luft/Sauerstoffgemischs aus dem Zufuhrkanal (14) verhindert,
   - einen Abluftkanal (15) für das Luft/$CO_2$-Gemisch,
   - ein Filterelement (7) des Luft/$CO_2$-Gemisches in Fluidverbindung mit dem Plenum (6), und
   - eine Steuereinheit (12) zur Regulierung von zumindest der Sauerstoffsättigung und der Kohlendioxidkonzentration im Inneren des Atemschutzhelms (13);

   das künstliche Beatmungssystem (100, 200) ist **dadurch gekennzeichnet, dass**:

   - die Strömungsrate des Luft/$CO_2$-Gemisches im Abluftkanal (15) durch ein drittes Steuerventil (8) geregelt wird,
   - der Zufuhrkanal (14) und der Abluftkanal (15) in einem ersten Gehäuse (16) integriert sind, das mit einem zweiten Gehäuse (17) im Inneren des Atemschutzhelms (13) verbunden ist,
   - wobei in dem ersten Gehäuse (16) und dem zweiten Gehäuse (17) die beiden Ströme mittels eines Trennseptums (20) getrennt bleiben, und
   - das zweite Gehäuse (17) eine erste Vielzahl von Löchern (17') aufweist, die es dem Luft-/Sauerstoffgemisch ermöglichen, in den Atemhelm (13) zu strömen, und eine zweite Vielzahl von Löchern (17") für den Eintritt der abgesaugten $CO_2$-reichen Luft in das zweite Gehäuse (17).

2. Künstliches Beatmungssystem (100, 200) nach Anspruch 1, wobei das erste Gehäuse (16) in eine Kupplung (18) integriert ist.

3. Künstliches Beatmungssystem (100, 200) nach Anspruch 2, wobei die Kupplung (18) mit Sensoren (19) ausgestattet ist, die mit der Steuereinheit (12) in Verbindung stehen.

4. Künstliches Beatmungssystem (100, 200) nach einem der vorhergehenden Ansprüche, wobei das Plenum (6) eine Ultraviolettlichtvorrichtung (11) zum Sterilisieren der Luft/Sauerstoff-Gemisch.

5. Künstliches Beatmungssystem (100, 200) nach einem der vorhergehenden Ansprüche, wobei:

   - das Saugmittel ein Ejektor (5) ist, der von einem Hauptstrom gespeist wird, der aus einem ersten Kanal (3') kommt, der sich stromabwärts des ersten Steuerventils (2) befindet, und von einem Sekundärstrom, der aus einem zweiten Kanal (3") kommt, der parallel zum ersten Kanal (3') ist und sich ebenfalls stromabwärts des ersten Steuerventils (2) befindet, und
   - eine kalibrierte Öffnung (3), die zwischen den beiden Kanälen (3', 3") angeordnet ist, erzeugt einen Druckunterschied zwischen dem Sekundärstrom und dem Hauptstrom.

6. Künstliches Beatmungssystem (200) nach einem der Ansprüche 1 bis 4, wobei das Saugmittel (5) ein elektrischer Ventilator (25) ist, der einen Überdruck zur Belüftung im Inneren des Atemhelms (13) und einen Unterdruck zum Ansaugen der vom Atemhelm (13) eingeatmeten Luft erzeugt.

7. Künstliches Beatmungssystem (100, 200) nach Anspruch 1, wobei dem LuftSauerstoff-Gemisch ein zweites Luft-/Medizingasgemisch hinzugefügt wird und das künstliche Beatmungssystem (100, 200) einen zweiten Tank (1A), ein erstes Steuerventil (2A) zum Mischen der Luft und des Medizingases, eine zweite kalibrierte Öffnung (3A) zum Erzielen eines Druckabfalls und ein zweites Steuerventil (4A) zum Regeln der Strömungsrate dieses zweiten Luft-/Medizingasgemisches umfasst.

**Revendications**

1. Système de ventilation artificielle (100, 200) comprenant:

   - un casque respiratoire (13),
   - des moyens d'aspiration (5, 25) qui aspirent l'air de l'environnement extérieur,
   - un réservoir (1) destiné à contenir de l'oxygène sous pression,
   - une première vanne de régulation (2) qui régule le mélange air/oxygène,
   - une seconde vanne de régulation (4) qui régule le débit du mélange air/oxygène,
   - un plénum (6) destiné à contenir le mélange air/oxygène sortant des moyens d'aspiration (5),
   - un conduit d'alimentation (14) qui permet au mélange air/oxygène d'atteindre le casque respiratoire (13),
   - un premier clapet anti-retour (9) qui empêche le reflux du mélange air/oxygène depuis le conduit d'alimentation (14),
   - un conduit d'évacuation (15) du mélange air/CO2
   - un élément filtrant (7) du mélange air/CO2 en communication fluidique avec le plénum (6), et
   - une unité de commande (12) pour réguler au moins la saturation en oxygène et la concentration en dioxyde de carbone à l'intérieur du casque respiratoire (13);

   le système de ventilation artificielle (100, 200) étant **caractérisé en ce que**:

   - le débit du mélange air/CO2 dans le conduit d'évacuation (15) est régulé par une troisième vanne de régulation (8),
   - le circuit d'alimentation (14) et le circuit d'évacuation (15) sont intégrés dans un premier boîtier (16) qui se raccorde à un deuxième boîtier (17) à l'intérieur du casque respiratoire (13),
   - dans lequel entre le premier boîtier (16) et le second boîtier (17), les deux flux restent séparés au moyen d'un septum de séparation (20), et
   - le second boîtier (17) comprend une première pluralité de trous (17') qui permet au mélange air/oxygène de circuler à l'intérieur du casque respiratoire (13) et une seconde pluralité de trous (17") pour l'entrée de l'air riche en CO2 évacué dans le second boîtier (17).

2. Système de ventilation artificielle (100, 200) selon la revendication 1, dans lequel le premier boîtier (16) est intégré dans un raccord (18).

3. Système de ventilation artificielle (100, 200) selon la revendication 2, dans lequel le raccord (18) est équipé de capteurs (19) en communication avec l'unité de commande (12).

4. Système de ventilation artificielle (100, 200) selon l'une quelconque des revendications précédentes, dans lequel le plénum (6) comprend un dispositif à lumière ultraviolette (11) pour stériliser le mélange air/oxygène.

5. Système de ventilation artificielle (100, 200) selon l'une quelconque des revendications précédentes, dans lequel:

   - le moyen d'aspiration est un éjecteur (5) alimenté par un flux principal provenant d'un premier conduit (3'), situé en aval de la première vanne de régulation (2), et par un flux secondaire provenant d'un deuxième conduit (3"), parallèle au premier conduit (3') et également situé en aval de la première vanne pilote (2), et
   - un orifice calibré (3) interposé entre les deux conduits (3', 3") crée une différence de pression entre le flux secondaire et le flux principal.

6. Système de ventilation artificielle (200) selon l'une quelconque des revendications 1 à 4, dans lequel le moyen d'aspiration (5) est un ventilateur électrique (25) qui crée une surpression pour la ventilation à l'intérieur du casque respiratoire (13) et une dépression pour l'aspiration de l'air respiré par le casque respiratoire (13).

7. Système de ventilation artificielle (100, 200) selon la revendication 1, dans lequel un deuxième mélange air/gaz médical est ajouté au mélange air/oxygène et le système de ventilation artificielle (100, 200) comprend un deuxième réservoir (1A), une première vanne de régulation (2A) pour mélanger l'air et le gaz médical, un deuxième orifice calibré (3A) pour obtenir une perte de charge et une deuxième vanne de régulation (4A) qui régule le débit de ce deuxième mélange air/gaz médical.

**Fig. 1**

**Fig. 2**

**Fig. 3**

**Fig. 4**

**Fig. 5a**

**Fig. 5b**

**Fig. 5c**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019167928 A